# EUROPEAN PATENT APPLICATION

(11) **EP 3 501 592 A1**
(43) Date of publication of application: **26.06.2019**
(21) Application number: 17841113.8
(22) Date of filing: 18.08.2017
(51) Int. Cl.: A61M 37/00

(54) **NON-INVASIVE DELIVERY METHOD**

(30) Priority: 19.08.2016 CN 201610698366
(71) Applicant: Ong, Tze Guan, Beijing 100025 (CN)
(72) Inventor: Ong, Tze Guan, Beijing 100025 (CN)
(74) Representative: Mewburn Ellis LLP
(86) International application number: PCT/CN2017/098037
(87) International publication number: WO 2018/033141

(57) **Abstract**

Provided herein is a non-invasive transportation method, relating to the field of transdermal transportation, which is capable of improving absorption of active ingredients by skin. The non-invasive transportation method comprises: applying needlelike crystals onto the skin, wherein the needlelike crystals form a micro-channel array in epidermis. The method is applicable to transdermal transportation of active ingredients, and more commonly used in cosmetic methods.

## Description

The present application claims priority to Chinese Patent Application No. 201610698366.X, filed with the China Patent Office on August 19, 2016 and entitled "NON-INVASIVE TRANSPORTATION METHOD," the entirety of which is hereby incorporated by reference.

### TECHNICAL FIELD

The present invention relates to the field of transdermal transportation and in particular to a non-invasive transportation method. The method can be used in cosmetology and dermal regeneration.

### BACKGROUND

As the generation rate of important compounds required by the skin decreases with age, the skin suffers from loss of collagen and elastic fibers, causing functional decline of dermal structure of the skin. Proper stimuluses and active substances are thus necessary to keep an optimal dermal regeneration.

In the case where active substances are used, absorption of the active substances by the skin usually is limited due to the presence of the epidermal stratum corneum. The epidermal stratum corneum, a protective barrier formed by superficial cells, prevents macromolecular substances and organic substances from entering into the skin and being absorbed by human body. Many active substances cannot smoothly pass through the epidermis into the dermis or deeper into the blood vessels, resulting in poor regeneration of the dermis by applying active substances.

To solve this problem, invasive or non-invasive transdermal transportation techniques have been developed.

In the invasive transdermal transportation techniques, the epidermis is punctured by a device with small needles, providing a resulted punctured channel through which the active substances enter into the skin. Another way to use the invasive transdermal transportation techniques is to inject the active substances directly into the dermis.

These invasive transdermal transportation techniques bring good technical effects, and yet at the same time, damage to the skin. If not treated properly, the damage may cause complications.

In the non-invasive transdermal transportation techniques, various methods are used to promote the penetration of the active substances into the dermis to a certain extent through the natural passage of the stratum corneum and sweat glands as well as hair follicles. Examples of the non-invasive transdermal transportation techniques include, but are not limited to, iontophoresis, electrophoresis, electroporation, water jetting, air jetting, micro-needle based devices, ultrasound, laser dermabrasion and other dermabrasion treatments and the like.

Although the non-invasive transdermal transportation techniques show deeper penetration of the active substances into the dermis, they provide poor technical effects as compared with the invasive transdermal transportation techniques because, in the non-invasive transdermal transportation techniques, the active substances need to penetrate through the stratum corneum, which, however, prevents the penetration of the active substances.

### SUMMARY OF THE INVENTION

One of the main objectives of the embodiments of the present invention is to provide a non-invasive transportation method which will greatly improve the efficacy of the transdermal transportation with respect to the existing non-invasive transdermal transportation techniques.

To achieve this objective, the embodiments of the present invention employ the following technical solutions.

A non-invasive transportation method, comprising:
applying needlelike crystals onto skin,
wherein the needlelike crystals form a micro-channel array in epidermis layer, whereby active ingredients are transported into the skin through the micro-channel array.

Optionally, said applying needlelike crystals onto the skin comprises:
mixing the needlelike crystals with the active ingredients prior to applying them onto the skin; or
applying the needlelike crystals onto the skin prior to applying the active ingredients onto the skin.

Optionally, the method further comprises:
using additional transdermal transportation methods to facilitate transportation of the active ingredients into the skin,
wherein the additional transdermal transportation methods include, but are not limited to, iontophoresis, electrophoresis, electroporation, water jetting, air jetting, micro-needle based devices, ultrasound, laser dermabrasion and other dermabrasion treatments.

Optionally, the method further comprises:
pressing or kneading the skin to facilitate absorption of the active ingredients.

Optionally, the method may be used in cosmetology or regeneration techniques.

By applying crystals with needlelike microstructure on the face to form a micro-channel array, the non-invasive transportation method provided in the embodiments of the present invention is able to improve the penetration of active substances through skin barrier, and deeply nourish the skin. Additionally, after penetrating into the skin, the micro-needle structure of the crystals will stimulate the skin's vitality, enabling the production of substances such as collagen by the skin *per se*, and thereby promoting skin regeneration. Finally, the crystals in the skin will be degraded, due to the naturopathy of the skin, and absorbed by the skin.

### BRIEF DESCRIPTION OF THE DRAWINGS

In order to illustrate the technical solutions of the embodiments of the present invention or of the prior art more clearly, the accompanying drawings to be used to explain the embodiments of the present invention or the prior art will be briefly described below. Apparently, the accompanying drawings described below only represent some of the embodiments of the present invention, and a person of ordinary skill in the art can obtain other drawings based on these drawings without paying any creative effort.
Fig. 1 shows a sectional view of the skin structure.
Fig. 2 shows a picture of the finished product of a roller-type or a pressing-type micro-needle in the prior art.
Fig. 3 shows comparison among the roller-type micro-needle, the pressing-type micro-needle, and Embodiment 1 of the present application, when applied to the skin.
Fig. 4 shows comparison among the roller-type micro-needle, the pressing-type micro-needle, and Embodiment 1 of the present application, after applied to the skin.
Fig. 5 shows needlelike crystals suspended in homogeneous liquid, observed under microscope (100X).
Fig. 6 shows a side view and a sectional view of the needlelike crystals.
Fig. 7 shows the needlelike crystals observed under optical microscope (10X x 40X).

### Reference numerals:

1- epidermis layer;
11- stratum corneum;
12- stratum lucidum;
13- stratum granulosum;
14- stratum spinosum;
15- stratum basale;
2- dermis;
21- tactile corpuscle;
22- capillary;
23- dermal papilla;
24- arrector pili muscle;
25- sebaceous gland;
3- subcutaneous tissue;
31- hair shaft;
32- sweat gland pore;
33- sweat gland duct;
34- sweat gland;
35- adipose tissue;
36- arteriole;
37- venule;
38- hair papilla; and
39- corpuscle.

### DETAILED DESCRIPTION OF THE INVENTION

The technical solutions of the embodiments of the present invention will be clearly and completely described hereafter with reference to the accompanying drawings involved in the embodiments of the present invention.

The non-invasive transportation method provided by the embodiments of the present invention will be described below with reference to the accompanying drawings.

The non-invasive transportation method provided by the present invention uses crystals having a needlelike microstructure. After being applied onto the skin, the needlelike crystals will form in the epidermis layer a micro-channel array through which the active ingredients are able to be transported into the skin.

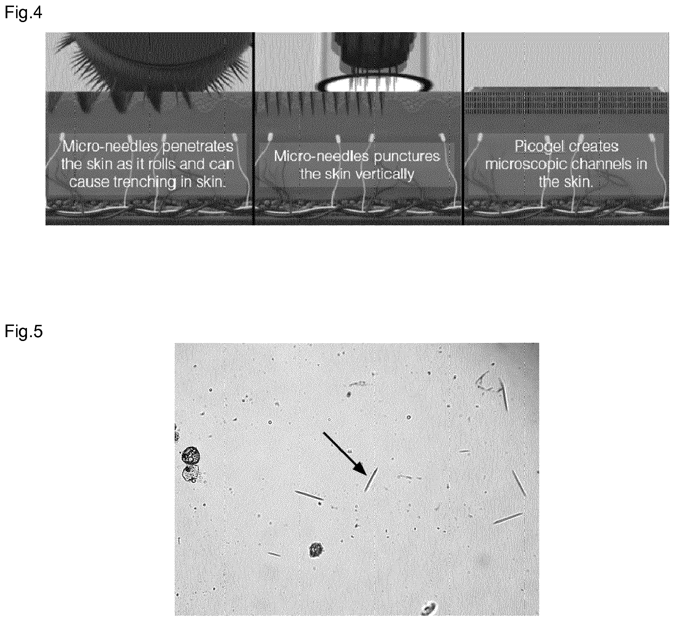

The crystals having a needlelike microstructure are invisible to the naked eyes, and suspended in a homogeneous liquid. Fig. 5 shows the microstructure of the crystals observed under microscope at 100X magnification.

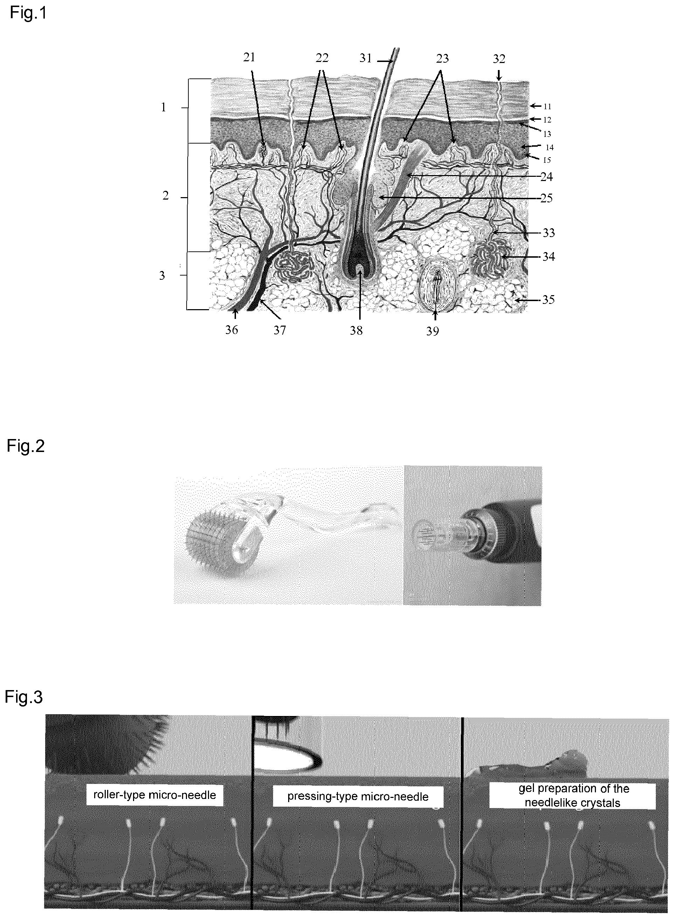

Referring to Fig. 1, it can be seen that the epidermis layer 1 of skin is composed of stratum corneum 11, stratum lucidum 12, stratum granulosum 13, stratum spinosum 14 and stratum basale 15; and the dermis 2 of the skin includes tactile corpuscles 21, capillaries 22, dermal papillae 23, arrector pili muscles 24, sebaceous glands 25 and sweat gland ducts 33.

Depending on the age, gender, photo-aging, different parts of the body and the like of an individual, the thickness of the skin (the epidermis 1 and the dermis 2) varies widely. Taking the facial skin as an example, referring to Table 1, the facial skin (the epidermis 1 and the dermis 2) has a depth of between 0.5 mm to 1.5 mm; the corresponding facial epidermis 1 has a thickness of between 0.05 mm to 0.2 mm; and the stratum corneum 11 correspondingly has a thickness of between 0.015 mm to 0.02 mm. The stratum corneum 11 is the main skin barrier. For the penetration of important active substances, certain measures must be taken to allow the active substances to easily pass through the stratum corneum.

**Table 1. The average of the measured skin thickness**

| Site | Subject A (mm) | Subject B (mm) | Subject C (mm) |
|---|---|---|---|
| Upper lip | 0.68±0.09 | 1.01±0.01 | 0.79±0.16 |
| Lower lip | 0.78±0.21 | 0.83±0.07 | 0.85±0.15 |
| Philtrum | 0.90±0.08 | 0.83±0.09 | 0.76±0.09 |
| Jaw | 1.06±0.10 | 1.24±0.05 | 1.06±0.11 |
| Upper eyelid | 0.41±0.13 | 0.40±0.06 | 0.32±0.05 |
| Lower eyelid | 0.84±0.06 | 1.04±0.04 | 0.57±0.05 |
| Forehead | 0.90±0.13 | 1.16±0.11 | 1.04±0.04 |
| Right cheek | 1.04±0.10 | 1.07±0.06 | 1.11±0.11 |
| Left cheek | 1.11±0.09 | 1.20±0.09 | 1.20±0.04 |
| Malar eminence | 0.97±0.07 | 1.62±0.05 | 0.57±0.04 |
| Submentum | 1.06±0.04 | 0.97±0.05 | 0.65±0.09 |
| Nasal cavity | 1.37±0.14 | 1.17±0.09 | 1.11+0.06 |
| Nasal dorsum | 0.60±0.06 | 0.79±0.06 | 0.81±0.09 |
| Right neck | 0.55±0.09 | 0.25±0.04 | 0.77±0.07 |
| Left neck | 0.38±0.04 | 0.43±0.03 | 0.80±0.05 |
| Subject A was an 82-year-old female subject, subject B was a 51-year-old female subject, and subject C was a 78-year-old male subject. | | | |

After being applied onto the skin, the crystals with needlelike structure form in the epidermis layer 1 of the skin a dense and uniform micro-channel array having a depth of 0.02 mm to 0.5 mm. Preferably, with the aid of additional means, the depth of the micro-channel array can be further deepened, for example, up to 0.1 mm to 0.8 mm. The micro-channel array penetrates through the stratum corneum 11 which is the main component of the skin barrier, thereby promoting further penetration of the active ingredients into the dermis 2 through the skin barrier. It should be noted that if the present transportation method is performed in conjunction with other means such as massage, the micro-channel array can be formed at a deeper level. That is, the array of the crystals can be deepened by massage.

After creating the micro-channel array, the crystals usually will be completely absorbed by the body through a natural defense mechanism, within 12 hours to 48 hours. Preferably, the crystals will be completely absorbed by the skin within 6 hours to one day. No damage to the skin is occurred.

The specific composition of the crystals is not specifically limited in the present application. Any crystals that can be absorbed by the skin within 2 to 14 days, have a needlelike microstructure and produce no harm to the skin, may be used.

Such crystals can be extracted from nature, for example, from plants. Of course, it is also possible to artificially synthesize such crystals that have a needlelike structure and can be eventually degraded and absorbed by the skin. In this regard, the present application will make no specific limitation.

The plants from which the crystals can be extracted are preferably plant varieties from Araceae. The needlelike crystals present in such plant varieties mainly function to cause a sensation of pain from gnawing to bugs or other organisms that gnaw their roots, stems, leaves or other parts, so as to provide a protection to the plants themselves. Such needlelike crystals have no toxic or side effect, and can be degraded and absorbed by an organism.

The size of the needlelike crystals is generally in µm and nm scale. For example, the crystals may have a width of between 0.3 µm to 8 µm, and a length of between 20 µm to 600 µm. It depends on the sizes of the needlelike crystals obtained from nature or synthesized artificially. The present application will make no further limitation to the size, as long as the crystals are able to enter into the skin without causing any invasive injuries.

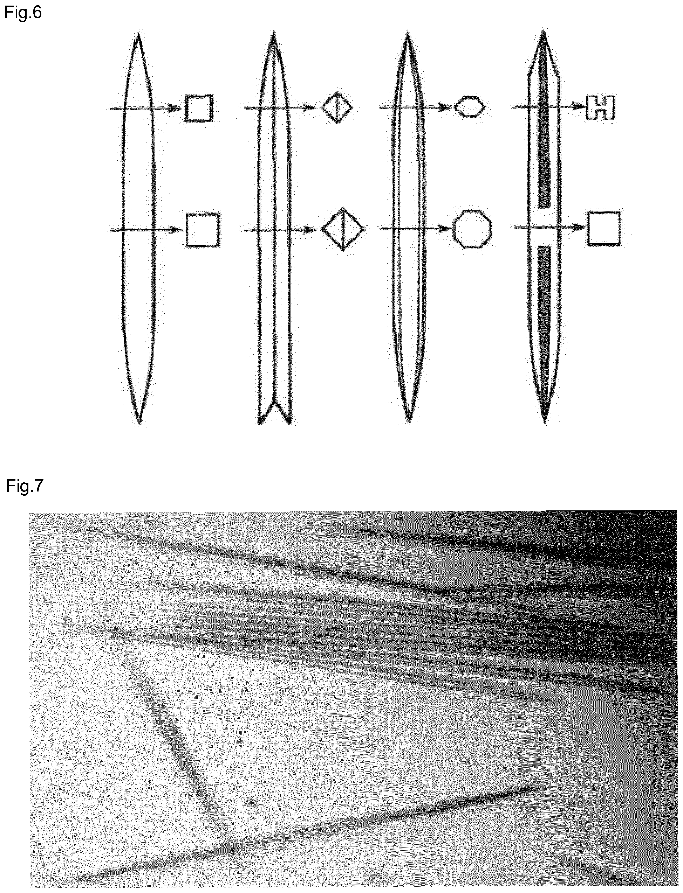

In order to show the microstructure of the needlelike crystals more vividly, in addition to Fig. 5 showing the needlelike crystals in the suspension observed under the 100X microscope, the present application also provides Fig. 7, showing calcium oxalate raphides extracted from roots of *Lasia spinosa* observed under optical microscope (10X x 40X).

Additionally, said needlelike shape just generally describes the shape of the crystals. Any elongated structure facilitating penetration into the skin can be used. For example, in the longitudinal direction, spindle shape or arrow shape is preferred. The cross-section, either hollow or solid, may be of a variety of geometric shapes, for example, a shape having smooth periphery such as a circle, an ellipse or the like; or a shape having angled periphery such as a square, a polygon, a triangle, elongated rectangles or the like. Due to the large aspect ratio of the needlelike crystals, the differences in the shapes of the cross-section will have little influence on the ability to penetrate into the skin, although it is preferable to have a cross-section in circle.

To better illustrate the possible micro-shapes the needlelike crystals may have, some specific shapes of the needlelike crystals will be specifically exemplified below. As shown in Fig. 6, the needlelike crystal of type 1 has a structure in spindle shape in the longitudinal direction, which is thick in the middle and tapered at both ends, and the structure has a cross-section in solid square shape. The needlelike crystal of type 2 has a structure in arrow shape in the longitudinal direction, which is protrude from the middle of the top end and sunk in the tail end, and the structure has a cross-section in hollow rhombus shape with an interlayer in the middle. The needlelike crystal of type 3 is in a spindle shape in the longitudinal direction, and it has a cross-section in hollow polygon shape. The needlelike crystal of type 4 has a structure in spindle shape in the longitudinal direction, and the structure has a cross-section in H shape in both the upper end and lower end, and hollow square shape in the middle.

In addition to single crystals, the needlelike crystals can also include needlelike crystal bundles formed by clustering and aggregation of multiple single crystals due to the synthesis processes and natural extraction processes. When in use, the needlelike crystal bundles, as a whole, enter into the skin.

In the existing non-invasive transportation methods, the active ingredients permeate into the skin mainly by virtue of osmosis, which, however, has very little effect, leaving most of the active ingredients blocked outside the skin barrier, and thereby resulting in the waste of the active ingredients. By using the present method, with the aid of the micro-channel array created by the needlelike crystals, the active ingredients are able to penetrate through the skin barrier, thus absorption of the active ingredients will be improved.

Compared with the existing invasive transportation methods, the crystals of the present application do not cause injuries to the skin, and they also have a much better homogeneous distribution on the surface of the skin as compared with the micro-needle method used in invasive cosmetology. Fig. 2 shows pictures of products of a roller-type micro-needle (left) and a pressing-type micro-needle (right) present in the prior art. Fig. 3 shows a schematic view of the existing invasive roller-type micro-needle (e.g., Derma roller-type micro-needle) and pressing-type micro-needle as well as the gel preparation of the non-invasive needlelike crystals of the present application, before applied on the skin. Fig. 4 shows a schematic view of the three after applied on the skin. Both the roller-type micro-needle and the pressing-type micro-needle caused injuries to the skin. Moreover, since the diameter of the needle tips in the existing micro-needle methods is approximately in millimeter scale and the distance between the needle tips is also in millimeter scale, the skin cannot get dense care. For example, for the pressing-type micro-needle, the size of the needle tips is large (0.5 mm to 1 mm) and the distance between the needle tips is also large (e.g., 0.3 mm), leaving large area of skin between the needle tips, and thus the skin cannot be covered completely and uniformly. However, the needlelike crystals of the present application do not cause injuries to the skin, and, due to the effect of the microscopic size, they are able to form very dense micro-channels. It should be noted that, in order to display the arrangement of the needlelike crystals more intuitively, the needlelike crystals are plotted in a tangible dense arrangement. In fact, these needlelike crystals are invisible to naked eyes, and they can only be observed with the help of a microscope.

In addition to improving the absorption of the active ingredients, during penetration into the skin, the emergence of the needlelike crystals also stimulate the immune system of the skin, enabling production of new collagen and fibers by the skin, and thereby improving the dermal structure and increasing the flexibility and elasticity of the skin.

The active ingredients can be mixed with the needlelike crystals and then applied onto the skin; or, the active ingredients can be applied after applying the needlelike crystals. A homogeneous liquid will be resulted after mixing with the active ingredients. The needlelike microstructure of the crystals is invisible to the naked eyes. For the kinds of the active ingredients, the present application will make no specific definition. For example, the active ingredients can be an active ingredient for cosmetic use or for medical use. The active ingredients for cosmetic use can be classified based on their effects such as moisturizing, whitening, anti-aging or the like. Specifically, the active ingredients can be a hyaluronic acid, a collagen, a vitamin C, a vitamin B3, an aloe, an arbutin, a linolenic acid, a botulinum toxin, a hepatic cell, a sheep placenta extract, a human placenta extract, various enzymes or the like. The active ingredients for medical use can be any existing medicines transportable by using a transdermal transportation system. The method of the present invention can be used alone or in combination with an existing transdermal transportation system so as to achieve better effects.

In addition, for the dosage form of the product, the crystals can be formed as a liquid dosage form, a paste dosage form, a gel dosage form or the like by using the existing processes and active ingredients.

After being applied onto the skin, the absorption of the needlelike crystals can be promoted by means such as manual patting, massaging, kneading, pressing or the like.

In addition, as a preferred embodiment of present invention, after the needlelike crystals and active ingredients being applied, additional transdermal transportation techniques, for example, iontophoresis, electrophoresis, electroporation, water jetting, air jetting, micro-needle based devices, ultrasound, laser dermabrasion and other dermabrasion treatments and the like, may be used to further enhance the absorption of the needlelike crystals.

Both the iontophoresis and the electrophoresis methods are based on the effect of electric field. The electric field acts on charged particles so as to non-invasively transport the active substances into the skin.

The effect of the electrophoresis generally can last for 5 minutes to 2 or 3 hours; preferably 5 minutes to 60 minutes, further preferably 10 minutes to 30 minutes, and further preferably 10 minutes to 20 minutes. Comparing with the prior art using the electrophoresis technique alone, the electrophoresis technique used in the present invention, with the aid of the micro-channel array created by the needlelike crystals, can rapidly and greatly promote the absorption of the active ingredients.

The electroporation is a cell introduction method. The cell membrane of the dermis is mainly composed of phospholipid molecules. The spatial structure of the phospholipid bilayer can be instantaneously opened by appropriate electric current and then recover, allowing easy entry of the active substances when the spatial structure is open. This method maintains the integrity of the cell membrane, and thus is a good non-invasive cell introduction method. The transportation method of the present invention, used in conjunction with the electroporation method, is able to exhibit synergistic effect in absorbing the active substances. With regard to the water jetting method, high-pressure water is used to impact the facial skin, so as to better penetration of the active substances into the skin through sweat glands, and to thin the thickness of the stratum corneum at the same time, thereby facilitating entry of the active substances. The air jetting method, similar in principles with the water jetting method, uses high-pressure air to act on the surface of the skin so as to promote entry of the active substances. In the ultrasound method, a liquid is placed on the face and then ultrasonically treated so that microbubbles will be generated from the liquid. The microbubbles will function to promote entry of the active substances into the skin at the time they are burst. The laser dermabrasion method and other dermabrasion treatments mainly focus on thinning the stratum corneum so as to further promote entry of the needlelike crystals and the active substances. All the above-described methods can be used at the same time as the needlelike crystals and/or the active ingredients are applied, so as to jointly provide a synergistic effect. Based on the above-described principles of the present application, the non-invasive transportation method provided by the present application is useful in various cosmetic methods. Optionally, if the active ingredient is a medical ingredient, the non-invasive transportation method can also be used in a method of treatment. In conclusion, the present application aims to provide a non-invasive transportation method which is useful in various fields where it is necessary to promote the transdermal absorption of the substance through the skin.

To better illustrate the non-invasive transportation method provided by the present application, the present application will be described with specific examples.

### Example 1

1. Cosmetics, grease and dust were cleaned off the subjects' face with cleansing emulsion and cleaning gel.
2. Active ingredients were applied onto the subject's face for multiple times, 1 ml each time, specifically:
   a. 1 ml of the active ingredients were applied onto and uniformly covered on the facial skin;
   b. another 1 ml of the active ingredients were applied onto and uniformly covered on the facial skin as the previously applied active ingredients on the facial skin dried due to absorption and evaporation;
   c. steps a and b were repeated until a total of 5 ml of the active ingredients were used; and
   d. the time length from the initial application to the last application of the active ingredients was recorded.

### Comparative Example 1

On the fifth day after receiving the treatments of Example 1, the subjects received the treatments of Comparative Example 1, so as to avoid interference from the previous trial to the following trial and ensure the conditions of the skin in Example 1 and Comparative Example 1 were the most similar.
1. Cosmetics, grease and dust were cleaned off the subjects' face with cleansing emulsion and cleaning gel.
2. 1 ml of the needlelike crystals was applied onto the face, which was then massaged hard for 1 minute.
3. Active ingredients were applied onto the subject's face for multiple times, 1 ml each time, specifically:
   a. 1 ml of the active ingredients were applied onto and uniformly covered on the facial skin;
   b. another 1 ml of the active ingredients were applied onto and uniformly covered on the facial skin as the previously applied active ingredients on the facial skin dried due to absorption and evaporation;
   c. steps a and b were repeated until a total of 5 ml of the active ingredients were used; and
   d. the time length from the initial application to the last application of the active ingredients was recorded.

Note: the active ingredients used in the above-described experiments were stem cell extracts.

The total time required to use up all of the active ingredients by each of the five subjects is listed in Table 2.

**Table 2. Time for absorption of the active ingredients**

| Example 1 | | Comparative Example 1 | |
|---|---|---|---|
| Subject No. | Absorption time (min) | Subject No. | Absorption time (min) |
| Subject 1 | 18 | Subject 1 | 12 |
| Subject 2 | 16 | Subject 2 | 12 |
| Subject 3 | 16 | Subject 3 | 10 |
| Subject 4 | 20 | Subject 4 | 13 |
| Subject 5 | 18 | Subject 5 | 10 |
| Total absorption time | 88 | Total absorption time | 57 |
| Average absorption time | 17.6 | Average absorption time | 11.4 |

As can be seen from Table 2, subjects, treated with the needlelike crystals, significantly shortened the absorption time of the active ingredients, by about 35.2%, suggesting improvement on the absorption ability to the active ingredients by the skin.

### Example 2

1. Cosmetics, grease and dust were cleaned off the subjects' face with cleansing emulsion and cleaning gel.
2. A reagent bottle containing 20 ml of the active ingredients was connected to the roller head of a TMT device.
3. The TMT device was turned on, and its power was set at 80%.
4. The rolling actuator annularly acted on the whole facial skin of the subject.
5. The time period, from the initial application of the active ingredients till the same were completely used up, was recorded.

### Comparative Example 2

The subjects were treated by steps to be described in the comparison example 2 five days later after the treatment in Embodiment 2, to avoid the interference of the previous test to the next test and ensure the skin condition in Embodiment 2 is most approximate to that in comparison example 2.
1. Cosmetics, grease and dust were cleaned off the subjects' face with cleansing emulsion and cleaning gel.
2. 2ml of the needlelike crystals were applied onto the face, which was then massaged hard for 1 minute.
3. A reagent bottle containing 20 ml of the active ingredients was connected to the rolling actuator of a TMT device.
4. The TMT device was turned on, and its power was set at 80%.
5. The rolling actuator annularly acted on the whole facial skin of the subject.
6. The time period, from the initial application of the active ingredients till the same were completely used up, was recorded.

Note: the active ingredients used in the above-described experiments were stem cell extracts. The TMT device, obtained from Mesoestetic, is an electrophoresis plus electroporation multifunctional device.

The total time required to use up all of the active ingredients by each of the five subjects is listed in Table 3.

**Table 3. Time for absorption of the active ingredients**

| Example 2 | | Comparative Example 2 | |
|---|---|---|---|
| Subject No. | Absorption time (min) | Subject No. | Absorption time (min) |
| Subject 1 | 42 | Subject 1 | 18 |
| Subject 2 | 46 | Subject 2 | 23 |
| Subject 3 | 38 | Subject 3 | 19 |
| Subject 4 | 34 | Subject 4 | 14 |
| Subject 5 | 45 | Subject 5 | 21 |
| Total absorption time | 205 | Total absorption time | 95 |
| Average absorption time | 41 | Average absorption time | 19 |

As can be seen from Table 3, subjects, treated with the needlelike crystals, significantly shortened the absorption time of the active ingredients, by about 53.7%, suggesting improvement on the absorption ability to the active ingredients by the skin.

Moreover, based on the comparison between Example 1 and Example 2, it is found that more active ingredients were able to be absorbed within nearly the same time period by using the needlelike crystals in combination with the employment of electrical introduction technique(s). Thus, synergistic effects can be achieved by using the needlelike crystals in combination with existing common transportation method(s).

The foregoing description merely illustrates specific embodiments of the present invention, which does not intend to limit the protection scope of the present invention. Based on the technical scope disclosed in the present invention, any person of ordinary skill in the art can easily conceive of variations or alternatives, which should be included within the protection scope of the present invention. Accordingly, the protection scope of the present invention shall be defined by the claims.

## Claims

1. A non-invasive transportation method, **characterized in** comprising:
applying needlelike crystals onto skin,
wherein the needlelike crystals form a micro-channel array in epidermis layer, whereby active ingredients are transported into the skin through the micro-channel array.

2. The non-invasive transportation method according to claim 1, **characterized in that** said applying needlelike crystals onto skin comprises:
mixing the needlelike crystals with the active ingredients prior to applying them onto the skin.

3. The non-invasive transportation method according to claim 1, **characterized in that** said applying needlelike crystals onto skin comprises:
applying the needlelike crystals onto the skin prior to applying the active ingredients onto the skin.

4. The non-invasive transportation method according to any one of claims 1 to 3, **characterized in** further comprising:
using additional transdermal transportation method(s) to facilitate transportation of the active ingredients into the skin,
wherein the additional transdermal transportation method(s) include, but are not limited to, iontophoresis, electrophoresis, electroporation, water jetting, air jetting, micro-needle based devices, ultrasound, laser dermabrasion and other dermabrasion treatments.

5. The non-invasive transportation method according to any one of claims 1 to 3, **characterized in** further comprising:
pressing or kneading the skin to facilitate absorption of the active ingredients.

6. The non-invasive transportation method according to claim 1, **characterized in that** the method is used in a cosmetic method.
